Europäisches Patentamt

European Patent Office

Office européen des brevets

(11) Veröffentlichungsnummer: **0 115 778**
**B1**

(19)

# EUROPÄISCHE PATENTSCHRIFT

(12)

(45) Veröffentlichungstag der Patentschrift:
16.09.87

(51) Int. Cl.⁴: **A 61 N  1/04,** A 61 N  1/36,
A 61 B  5/04

(21) Anmeldenummer: 84100160.5

(22) Anmeldetag: 09.01.84

(54) **Elektrode für medizinische Anwendungen.**

(30) Priorität: 11.01.83  DE 3300668

(73) Patentinhaber: **Siemens Aktiengesellschaft Berlin und
München, Wittelsbacherplatz 2, D-8000 München 2 (DE)**

(43) Veröffentlichungstag der Anmeldung:
15.08.84 Patentblatt 84/33

(72) Erfinder: **Mund, Konrad, Dr., Langenbrucker Weg 6,
D-8521 Uttenreuth (DE)**
Erfinder: **Freller, Helmut, Steinbergstrasse 34a,
D-8505 Röthenbach (DE)**
Erfinder: **Hörauf, Friedrich, Wilhelm-Marx-Strasse 26,
D-8500 Nürnberg (DE)**

(45) Bekanntmachung des Hinweises auf die Patenterteilung:
16.09.87 Patentblatt 87/38

(84) Benannte Vertragsstaaten:
DE FR GB IT NL

(56) Entgegenhaltungen:
EP - A - 0 043 461
EP - A - 0 054 781
EP - A - 0 064 289
DE - A - 2 165 622
DE - A - 2 613 072
DE - A - 2 702 240
FR - A - 2 225 179
US - A - 3 862 017
US - A - 4 281 669

IEEE TRANSACTIONS ON BIOMEDICAL ENGINEERING,
Band BME-29, Nr. 4, April 1982, New York H. LERNER, R.
ZAHRADNIK, M. BUCHBINDER "Miniature Implantable
Tantalum/Tantalum Oxide Stimulating Electrodes"
Seiten 290-292

## Beschreibung

Die Erfindung betrifft eine Elektrode für medizinische Anwendungen, insbesondere eine implantierbare Reizelektrode.

Elektroden für medizinische Anwendungen gelangen in Form von Effektoren und Sensoren zum Einsatz. Unter Effektoren werden dabei Elektroden verstanden, mit denen eine Reizwirkung ausgeübt wird. Sensoren sind Elektroden, mit denen gemessen wird. Beispiele für Effektoren sind Reizelektroden für Herzschrittmacher sowie Elektroden zur Reizung von Nerven und Muskeln. Als Sensoren kommen insbesondere Mikroelektroden zur Potentialaufnahme sowie EEG- und EKG-Elektroden in Betracht, d.h. Elektroden zur Messung von Gehirn- bzw. Herzströmen.

Aus der US-Patentschrift 4 281 669 ist eine Herzschrittmacher- bzw. Reizelektrode bekannt, welche aus einem (dichten) Metallsubstrat, d.h. aus einem Trägermaterial, und einer darauf befindlichen porösen Metallschicht besteht. Die Metallschicht setzt sich dabei aus Metallpartikeln zusammen, die an ihren Berührungspunkten miteinander sowie mit dem Substrat verbunden sind, so dass ein Netzwerk aus untereinander in Verbindung stehenden Poren gebildet wird. Metallsubstrat und Metallschicht können beispielsweise aus Titan bestehen.

Aus der EP-AI 0 054 781 ist eine implantierbare Elektrode mit einem porösen Sinterkörper aus elektrisch leitenden Teilchen bekannt. Diese Teilchen sind, wenigstens im Oberflächenbereich der Elektrode, an den nicht miteinander verbundenen Stellen mit einer dünnen Schicht aus einem Werkstoff versehen, welcher eine geringere elektrische Leitfähigkeit besitzt als der Werkstoff, aus dem die Teilchen bestehen. Ein Teil der Oberfläche des Sinterkörpers ist ferner mit einem elektrisch gut leitenden Metall versehen, das gegenüber Körperflüssigkeiten inert ist; als derartiges Metall findet Platin, Iridium oder eine Platin-Iridium-Legierung Verwendung. Bei der bekannten Elektrode bestehen die Teilchen des Sinterkörpers aus einem Metall, einer Metall-Legierung oder einer Metallverbindung eines Elements aus der Gruppe Tantal, Titan, Niob und Zirkonium, oder aus einer Legierung auf Kobalt-Chrom-Basis. Dabei wird Tantal bevorzugt, als Teilchenwerkstoff kann beispielsweise aber auch elektrisch leitfähiges $TiO_x$ verwendet werden. Durch Oxidation bzw. durch Anodisieren werden in diesem Fall dann im Oberflächenbereich der Elektrode die Teilchen an den Stellen, an denen sie nicht miteinander versintert sind, mit einer dünnen elektrisch nichtleitenden $TiO_2$-Schicht versehen. Als Teilchenwerkstoffe eignen sich bei der bekannten Elektrode ferner auch Nitride, Carbide und Carbonitride von Tantal, Titan, Niob oder Zirkonium. Wesentlich dabei ist aber, dass diese Werkstoffe den Sinterkörper, d.h. das Trägermaterial, bilden, und dass der Sinterkörper mit einer Schicht aus einem Werkstoff mit geringerer elektrischer Leitfähigkeit versehen ist, insbesondere mit einer Schicht aus anodisiertem Tantal bzw. $TiO_2$, d.h. mit einer Schicht aus Metalloxid.

Implantierbare Reizelektroden, beispielsweise für Herzschrittmacher, bestehen in den meisten Fällen aus einem Elektrodenschaft mit einer isolierten Kabelzuleitung und aus einem Elektrodenkopf zur Übertragung der Stimulationsimpulse, d.h. dem aktiven oder wirksamen Bereich der Elektrode. An derartige Elektroden werden im wesentlichen zwei Forderungen gestellt:

1. Das Elektrodenmaterial muss körperverträglich sein, d.h. die Bildung von Bindegewebsschichten sollte, wenn sie nicht ohnehin unterbleibt, sehr gering sein; die Dicke sollte auf jeden Fall unter 100 μm bleiben. Ausserdem soll die Reizschwelle weitgehend konstant bleiben.

2. An der Phasengrenze Elektrode/Körperflüssigkeit soll sich eine hohe Doppelschichtkapazität ausbilden, so dass der Polarisationsanstieg während der Reizimpulse (0,5 bis 1 ms, 1 Hz, 10 mA, 10 mm²) kleiner als 0,1 V bleibt.

Die geforderte hohe Doppelschichtkapazität wirkt sich bei Reizelektroden, und auch allgemein bei Effektoren, günstig aus, weil durch den aufgeprägten Strom nur geringe Potentialänderungen entstehen, elektrochemische Reaktionen mit der Körperflüssigkeit weitgehend unterbleiben und der Energieaufwand gering ist. Im Falle von Sensoren, bei denen nur ein kleiner Messstrom fliesst, erleichtert eine hohe Kapazität der Elektroden die Erfüllung der Anforderungen, die an die Eingangsimpedanz von Verstärkern zu stellen sind; ausserdem wird das Rauschen reduziert.

Die vorstehend genannten Forderungen werden in besonders hohem Masse von Elektroden erfüllt, bei denen der Elektrodenkopf, d.h. allgemein der aktive Bereich, aus Glaskohlenstoff besteht (siehe: DE-OS 2 613 072). Die hohe Doppelschichtkapazität von bis zu 0,1 F/cm² ν = 1 Hz) wird durch eine Aktivierung der Oberfläche des Glaskohlenstoffs erreicht, wobei eine dünne, fest haftende Schicht aus Aktivkohle, d.h. eine Oberfläche mit mikroporöser Struktur, erhalten wird.

Aktivierter Glaskohlenstoff stellt somit ein Elektrodenmaterial mit grosser Kapazität dar, das darüber hinaus auch eine gute Körperverträglichkeit aufweist, und kann deshalb die metallischen Materialien, wie Platin/Iridium für Reizelektroden, Platin und Wolfram für Mikroelektroden und Silber/Silberchlorid für EKG-Elektroden, ersetzen, welche eine Degenerierung des angrenzenden Gewebes bewirken. Bei Glaskohlenstoff andererseits können aber Probleme hinsichtlich der mechanischen Bearbeitung (bei der Herstellung) und der Kontaktierung auftreten, was wiederum bei Metallelektroden nicht der Fall ist.

Aufgabe der Erfindung ist es, die bislang bei Elektroden für medizinische Anwendungen hinsichtlich des Elektrodenmaterials auftretenden Probleme zu vermeiden und somit das Angebot an wirksamen und brauchbaren Elektroden zu verbreitern.

Dies wird erfindungsgemäss dadurch erreicht, dass die Elektrode aus einem elektrisch leitenden Trägermaterial besteht und im aktiven Bereich eine poröse Schicht aus einem Carbid, Nitrid oder Carbonitrid wenigstens eines der Metalle Titan, Vanadium, Zirkonium, Niob, Molybdän, Hafnium, Tantal oder Wolfram aufweist. Dabei soll der aktive Bereich wenigstens zum Teil eine derartige poröse Schicht aufweisen.

Die die Carbide, Nitride und Carbonitride bildenden Metalle sind sämtlich Elemente der vierten bis sechsten Nebengruppe des Periodensystems und zählen somit zu den sogenannten Übergangsmetallen. Carbide MeC und Nitride MeN der genannten Art (Me = Metall) sind beispielsweise TiC, TiN, ZrC, ZrN, TaC und TaN. Es handelt sich dabei im wesentlichen um die stöchiometrischen Verbindungen, Abweichungen vom stöchiometrischen Verhältnis können aber gegeben sein. Die Carbonitride weisen die Zusammensetzung $MeC_xN_{1-x}$ auf, wobei x einen Wert zwischen 0 und 1 annehmen kann; beispielhaft sei hierzu die Verbindung $TiC_{0,5}N_{0,5}$ genannt. In allen Fällen können auch «gemischte» Verbindungen vorliegen, d.h. Me kann für verschiedene Metalle stehen; eine derartige Verbindung ist beispielsweise das Carbid (W,Ti)C. Darüber hinaus können die genannten Verbindungen auch in Form von Gemischen zum Einsatz gelangen.

Bei der erfindungsgemässen Elektrode weist die poröse Schicht aus Metallcarbid, -nitrid oder -carbonitrid, die gut elektronisch leitend ist, im allgemeinen eine Dicke zwischen 1 und 100 μm auf; vorzugsweise liegt die Schichtdicke zwischen 5 und 50 μm. Dabei ergeben sich Doppelschichtkapazitäten von 10 $mF/cm^2$ bis zu 100 $mF/cm^2$. Aufgrund dieser hohen Kapazität und der sich daraus ergebenden niedrigen Polarisation sowie der durch eine gute Körperverträglichkeit bedingten Stabilität der Reizschwelle sind die erfindungsgemässen Elektroden mit Elektroden aus aktiviertem Glaskohlenstoff vergleichbar. Hinsichtlich der Kontaktierung weisen die erfindungsgemässen Elektroden, da sie aus elektrisch leitendem Material bestehen, gegenüber Glaskohlenstoff-Elektroden aber Vorteile auf.

Bei Herzschrittmachern dauern die einzelnen Reizimpulse 0,5 bis 1 ms. Dies bedeutet, dass innerhalb dieser kurzen Zeit der Strom weitgehend in die poröse Schicht eindringen muss, um die Kapazität so weit wie möglich zu nutzen. Dieses Ziel ist aber nur dann zu erreichen, wenn der ohmsche Widerstand des Elektrolyten im Porensystem ausreichend niedrig ist, wie dies bei der erfindungsgemässen Elektrode der Fall ist. Charakteristisch ist dabei das Produkt aus volumenbezogener Kapazität c, spezifischem Widerstand ϱ des Elektrolyten in den Poren und dem Quadrat der Dicke d der porösen Schicht. Die Grenzbedingung lautet: $2\pi \cdot v \cdot c \cdot \varrho \cdot d^2 < 1$.

Die poröse Carbid-, Nitrid- oder Carbonitridschicht befindet sich auf einem elektrisch leitenden Trägermaterial. Dieses Trägermaterial muss im wesentlichen blut- und gewebeverträglich, d.h. körperverträglich sein. Als Trägermaterial kommen bei der erfindungsgemässen Elektrode deshalb Metalle und Metallegierungen, wie Elgiloy und nichtrostender Stahl (sogenannter VA-Stahl), in Frage. Vorzugsweise werden Platin und Titan eingesetzt; daneben können aber auch andere Edelmetalle verwendet werden. Darüber hinaus kann das Trägermaterial beispielsweise auch aus mit Metall überzogenem Kunststoff bestehen. Bei der erfindungsgemässen Elektrode weist zumindest der aktive Bereich die dünne poröse Schicht auf. Gegebenenfalls können aber auch andere (metallische) Bereiche der Elektrode mit einer derartigen Schicht versehen sein.

Bei der erfindungsgemässen Elektrode kann sich zwischen dem Trägermaterial und der dünnen porösen Schicht vorteilhaft eine dichte Schicht befinden, die aus demselben Material besteht wie die poröse Schicht. So kann beispielsweise auf Titan als Elektrodenmaterial zunächst eine dichte Titannitridschicht und dann eine poröse Titannitridschicht angeordnet sein. Durch die zusätzliche dichte Schicht kann eine Mischpotentialbildung verhindert werden. Darüber hinaus entfällt hierbei auch das Erfordernis, dass das Trägermaterial körperverträglich sein muss. Die Dicke der dichten, d.h. nicht-porösen Schicht beträgt vorzugsweise zwischen 2 und 10 μm.

Die dünnen porösen Schichten werden vorzugsweise durch reaktives Ionenplattieren, d.h. durch physikalische Dampfabscheidung, auf dem als Substrat dienenden Trägermaterial, wie Titan und Platin, aufgebracht. Dazu wird beispielsweise mit einem Elektronenstrahlverdampfer aus einem Vorrat des carbid-, nitrid- bzw. carbonitridbildenden Metalls dieses Metall in einer stickstoff- und/oder methanhaltigen Atmosphäre (daneben ist beispielsweise Argon als Inertgas vorhanden) verdampft und dann wird auf dem Substrat die entsprechende Metallverbindung, d.h. das Carbid, Nitrid oder Carbonitrid, als dünne Schicht abgeschieden. Der $N_2$- bzw. $CH_4$-Partialdruck beträgt hierbei im allgemeinen etwa zwischen $5 \cdot 10^{-3}$ und $1 \cdot 10^{-1}$ mbar. Das reaktive Ionenplattieren kann aber auch mit einer Magnetronsputterquelle erfolgen, wobei die Reaktionsgasdrücke für $N_2$ bzw. $CH_4$ etwa um eine Grössenordnung niedriger liegen ($4 \cdot 10^{-4}$ bis $1 \cdot 10^{-2}$ mbar).

Bei der Herstellung einer Elektrode, bei der sich zwischen dem Trägermaterial und der porösen Schicht eine entsprechende dichte Schicht befindet, wird vorteilhaft so vorgegangen, dass während der Beschichtung des Substrats der $N_2$- und/oder $CH_4$-Partialdruck langsam erhöht wird, und zwar beispielsweise von einem Wert etwa zwischen $2 \cdot 10^{-3}$ und $8 \cdot 10^{-3}$ mbar auf einen Wert zwischen $5 \cdot 10^{-3}$ und $10^{-1}$ mbar. Bei einem derartigen Vorgehen bildet sich dann nämlich auf dem Substrat zuerst die dichte, d.h. nicht-poröse Schicht und nachfolgend die entsprechende poröse Schicht. Neben dem $N_2$- bzw. $CH_4$-Partialdruck ist die Bildung von dichten bzw. porösen Schichten im übrigen noch abhängig vom Ionenstrom der Gasentladung, die zwischen dem Substrat und dem Elektronenstrahlverdampfer gezündet wird.

Die erfindungsgemässe Elektrode eignet sich insbesondere für folgende Anwendungen:

- Reizelektroden

Das Porensystem der porösen Schicht erzeugt eine hohe Doppelschichtkapazität, die beispielsweise für Reizelektroden von implantierbaren Herzschrittmachern angestrebt wird, um den Energieaufwand gering zu halten.

- Mikroelektroden

Dies sind im einfachsten Fall dünne Drähte mit einem Durchmesser von weniger als 50 µm, welche zugespitzt sind. Derartige Elektroden sind vorteilhaft im wesentlichen vollständig mit einer dünnen porösen Schicht versehen. Dabei bildet sich dann beim Eintauchen in den Elektrolyt an der (aktiven) Elektrodenspitze die hohe Doppelschichtkapazität aus, während die Poren der porösen Schicht längs des Elektrodenschaftes, d.h. des Drahtes, einen guten Haftgrund für die Isolation darstellen.

- EEG- und EKG-Elektroden

Auch hierbei ist die erzielbare hohe Kapazität wichtig. Wesentlich ist aber auch, dass die poröse Schicht auf dem Trägermaterial abriebfest verankert ist und die Elektrode – nach entsprechender Reinigung – somit mehrfach verwendet werden kann.

- Gegenelektroden

Vorteilhaft ist es beispielsweise, wenn Reiz- und Gegenelektrode aus demselben Material bestehen, weil dann keine materialbedingten Potentialdifferenzen auftreten können.

In der gleichzeitig mit der vorliegenden Patentanmeldung eingereichten europäischen Patentanmeldung Nr. 84100158.9 ist eine bipolare Elektrode für medizinische Anwendungen, insbesondere eine implantierbare Herzschrittmacherelektrode, mit einem isolierten Leitersystem, mindestens einer aktiven und einer – im Abstand dazu entlang des Leitersystems angeordneten – indifferenten Elektrode beschrieben (siehe dazu EP-AI 0 116 280). In der ebenfalls gleichzeitig mit der vorliegenden Patentanmeldung eingereichten europäischen Patentanmeldung Nr. 84100159.7 ist ein Herzschrittmachersystem mit mindestens einer aktiven und einer indifferenten Elektrode beschrieben, wobei für die letztere insbesondere das Herzschrittmachergehäuse dient (siehe dazu EP-AI 0 117 972). Bei diesem Herzschrittmachersystem bzw. bei der bipolaren Elektrode der vorstehend genannten Art weist zumindest der aktive Bereich der indifferenten Elektrode eine Oberflächenschicht auf, die an der Phasengrenze zur Körperflüssigkeit eine hohe Doppelschichtkapazität besitzt. Als eine derartige Oberflächenschicht eignet sich insbesondere eine poröse Schicht aus einem Carbid, Nitrid oder Carbonitrid wenigstens eines der Metalle Titan, Vanadium, Zirkonium, Niob, Molybdän, Hafnium, Tantal oder Wolfram.

Anhand von Beispielen soll die Erfindung noch näher erläutert werden.

Bei den nachfolgend beschriebenen Untersuchungen wurden jeweils Elektroden verwendet, deren poröse Schicht eine Dicke von ca. 30 µm aufwies.

Zur Bestimmung der elektrochemischen Eigenschaften dienten beispielsweise mit Titannitrid beschichtete Titanbleche, die in einer Halbzellenanordnung mit 0,15 M NaCl als Elektrolyt untersucht wurden. Die Elektroden stellten dabei ein Potential $\gamma/H_{2\,rev} = 0,89$ V ein. Bei potentiodynamischer Belastung ergab sich zu Belastungsbeginn eine Doppelschichtkapazität von 68 mF/cm², die sich während einer Belastungsdauer von 88 h nicht änderte. Die Untersuchungen zeigten, dass bis zu einem Potential von 1,1 V keine Korrosion erfolgt, die Elektroden sind somit ausreichend stabil.

Zur Untersuchung der Körperverträglichkeit der Elektroden wurden sowohl Titanbleche mit einer schwarzen porösen TiN-Schicht als auch solche mit einer gelben dichten TiN-Schicht in den Oberschenkelmuskel von Katzen implantiert (Scheiben mit einem Durchmesser von 10 mm). Nach einer Implantationsdauer von 5 Wochen ergaben sich zwischen den verschiedenen Proben, d.h. bei passiv implantierten Elektroden, hinsichtlich des Bindegewebswachstums keine Unterschiede. Darüber hinaus betrug die Dicke der Bindegewebsschicht bei allen Proben weniger als 60 µm, d.h. es liegt eine nahezu ideale Gewebeverträglichkeit vor.

Aus Ti-Draht wurden Elektrodenköpfe in Form von Halbkugeln mit einem Durchmesser von 2 mm hergestellt. Diese Halbkugeln wurden mit porösem Titannitrid beschichtet, der Elektrodenschaft wurde mit einer Elgiloy-Wendel kontaktiert. Bei zur Implantation vorgesehenen Elektroden wird der Elektrodenschaft im übrigen stets mit einem geeigneten Material, wie Kunststoff, überzogen, so dass sich hierbei keine Probleme bezüglich der Körperverträglichkeit ergeben. Die Doppelschichtkapazität derartiger Reizelektroden, die potentiostatisch aus Impedanzmessungen ermittelt wurde, ergab sich – in 0,15 M NaCl – zu 21,5 mF/cm² bei $\gamma = 1$ Hz. Wegen der hohen Porosität der Schichten ist diese Kapazität aber bis zu Frequenzen von 10 Hz zugänglich.

Im Tierversuch (Implantation der Reizelektroden im Oberschenkelmuskel von Katzen) wurde eine Kapazität von 10,5 mF/cm² (bei $\gamma = 1$ Hz) ermittelt. Dieser Wert liegt – bedingt durch die Körperflüssigkeit – um ca. 50% niedriger als der Wert der in-vitro-Messungen (NaCl). Wesentlich ist dabei aber, dass sich die Kapazitätswerte auch nach einer Implantationsdauer von 42 Tagen nicht veränderten. Während dieser Zeit bildete sich eine dünne Bindegewebsschicht mit einer Dicke zwischen 30 und 60 µm, was erneut die gute Gewebeverträglichkeit unter Beweis stellt.

Die erfindungsgemässe Elektrode kann auch mehrere Bereiche aufweisen, die mit einer porösen Schicht versehen sind. Diese Bereiche wechseln sich dann mit Bereichen ab, welche keine poröse Schicht aufweisen. Durch eine derartige geometrische Anordnung kann erreicht werden, dass die Stromdichte in bestimmte Richtungen ansteigt.

## Patentansprüche

1. Elektrode für medizinische Anwendungen, insbesondere implantierbare Reizelektrode, dadurch gekennzeichnet, dass sie aus einem elektrisch leitenden Trägermaterial besteht und im aktiven Bereich eine poröse Schicht aus einem Carbid, Nitrid oder Carbonitrid wenigstens eines der Metalle Titan, Vanadium, Zirkonium, Niob, Molybdän, Hafnium, Tantal oder Wolfram aufweist.

2. Elektrode nach Anspruch 1, dadurch gekennzeichnet, dass die poröse Schicht eine Schichtdicke zwischen 1 und 100 µm, vorzugsweise zwischen 5 und 50 µm, aufweist.

3. Elektrode nach Anspruch 1 oder 2, dadurch gekennzeichnet, dass das Trägermaterial Titan oder Platin ist.

4. Elektrode nach einem der Ansprüche 1 bis 3, dadurch gekennzeichnet, dass sich zwischen dem Trägermaterial und der porösen Schicht eine dichte Schicht aus einem entsprechenden Material wie die poröse Schicht befindet.

5. Elektrode nach Anspruch 4, dadurch gekennzeichnet, dass die dichte Schicht eine Schichtdicke zwischen 2 und 10 µm aufweist.

6. Elektrode nach einem oder mehreren der Ansprüche 1 bis 5, dadurch gekennzeichnet, dass sie mehrere Bereiche mit einer porösen Schicht aufweist, wobei die Bereiche mit poröser Schicht durch Bereiche ohne poröse Schicht voneinander getrennt sind.

## Claims

1. An electrode for medical applications, particularly but not exclusively an implantable stimulating electrode, characterised in that it consists of an electrically conductive carrier material and in the active region is provided with a porous layer made of a carbide, nitride or carbonitride of at least one of the metals titanium, vanadium, zirconium, niobium, molybdenum, hafnium, tantalum, or tungsten.

2. An electrode as claimed in Claim 1, characterised in that the porous layer has a thickness of between 1 and 100 µm, preferably between 5 and 50 µm.

3. An electrode as claimed in Claim 1 or Claim 2, characterised in that the carrier material is titanium, or platinum.

4. An electrode as claimed in one of Claims 1 to 3, characterised in that a dense layer made of a material corresponding to that of the porous layer is present between the carrier material and the porous layer.

5. An electrode as claimed in Claim 4, characterised in that the dense layer has a thickness of between 2 and 10 µm.

6. An electrode as claimed in one or more of Claims 1 to 5, characterised in that it comprises a plurality of regions provided with a porous layer, the regions provided with a porous layer being separated from one another by regions not provided with a porous layer.

## Revendications

1. Electrode pour applications médicales, notamment électrode de stimulation susceptible d'être implantée, caractérisée en ce qu'elle est constituée en un matériau-support conducteur de l'électricité et comprend, dans la région active, une couche poreuse en carbure, nitrure ou carbonitrure d'au moins l'un des métaux que sont le titane, le vanadium, le zirconium, le niobium, le molybdène, l'hafnium, le tantale ou le tungstène.

2. Electrode suivant la revendication 1, caractérisée en ce que la couche poreuse a une épaisseur comprise entre 1 et 100 microns et, de préférence, comprise entre 5 et 50 microns.

3. Electrode suivant la revendication 1 ou 2, caractérisée en ce que le matériau-support est du titane ou du platine.

4. Electrode suivant l'une des revendications 1 à 3, caractérisée en ce qu'entre le matériau-support et la couche poreuse se trouve une couche dense en un matériau correspondant à celui de la couche poreuse.

5. Electrode suivant la revendication 4, caractérisée en ce que la couche dense a une épaisseur comprise entre 2 et 10 microns.

6. Electrode suivant l'une ou plusieurs des revendications 1 à 5, caractérisée en ce qu'elle comprend plusieurs régions ayant une couche poreuse, les régions ayant une couche poreuse étant séparées les unes des autres par des régions sans couche poreuse.